# EUROPEAN PATENT APPLICATION

(11) **EP 1 669 453 A1**
(43) Date of publication of application: **14.06.2006**
(21) Application number: 04787861.6
(22) Date of filing: 10.09.2004
(51) Int. Cl.: C12N 15/12, C07K 14/47, C07K 16/18, C12Q 1/04, C12Q 1/68, G01N 33/15, G01N 33/50

(54) **NOVEL NERVE STEM CELL MARKER**

(30) Priority: 12.09.2003 JP 2003321564
(71) Applicant: KANEKA CORPORATION, Osaka-shi, Osaka 530-8288 (JP); National Institute of Advanced Industrial Science and Technology, Tokyo 100-8921 (JP); Foundation for Biomedical Research and Innovation, Chuo-ku Kobe-shi Hyogo 6500047 (JP)
(72) Inventor: SYOFUDA, Tomoko, Biomedical R & I Center, Kobe-shi, Hyogo 650-0047 (JP); KANEMURA, Yonehiro, Amagasaki-shi, Hyogo 661-0974 (JP); MIYAKE, Jun, Amagasaki-shi, Hyogo 661-0974 (JP); NIWA, Hideo, Akashi-shi, Hyogo 674-0084 (JP); YAMASHITA, Kenji, Takamatsu-shi, Kagawa 761-8003 (JP)
(74) Representative: HOFFMANN EITLE
(86) International application number: PCT/JP2004/013221
(87) International publication number: WO 2005/030961

(57) **Abstract**

It is an object of the present invention to provide a method for detecting nerve stem cells/progenitor cells among a tissue or a mass of cells as constituted of a plurality of cell species, and a method for screening for a drug functioning as a nerve differentiation factor or the like by using the above-mentioned detection method.

It was found in this invention that the expression of the NC1 gene was high in undifferentiated nerve stem cells and said expression decreased with an advance of differentiation. Furthermore in this invention, by using the NC1 gene, the product thereof (NC1 protein) and the base sequences or the amino acid sequences thereof, antibodies or the like, it became possible to detect nerve stem cells/progenitor cells by way of various methods, so that a novel method for screening for a drug functioning as a nerve differentiation factor or the like was provided.

## Description

### TECHNICAL FIELD

This invention relates to a method for detecting nerve stem cells/progenitor cells among a tissue or a mass of cells as constituted of a plurality of cell species. The invention also relates to a method for screening for a drug or procedure, for example, by which nerve stem cells/progenitor cells are allowed to maintain their characters when a tissue or a mass of cells constituted of a plurality of cell species is subjected to a process or procedure for differentiation induction or by which nerve stem cells/progenitor cells can be induced in a mass of differentiated nerve cells.

### BACKGROUND ART

It has so far been considered that the central nerve system, once damaged, cannot be restored in its functions since neurons in charge of the nerve functions have themselves no division potential. In recent years, however, it has been revealed that nerve stem cells having both self-replicating ability and multipotency are present in the central nerve system of human adults and other animal adults as well, so that the so-called regenerative therapy, which comprises causing stem cells to differentiate into functioning cells or tissues to complement the damaged tissue or organ even in the central nerve system, has taken on a tough of real possibility (Reynolds, B. A. et al. (1992) "A multipotent EGF-responsive striatal embryonic progenitor cell produces neurons and astrocytes." J. Neurosci. 12:4565-74). The discovery of nerve stem cells has also made it possible to screen for drugs acting on nerve stem cells and causing them to differentiate into functioning nerve cells such as neurons or glial cells (Roy, N. et al. (2000) "In vitro neurogenesis by neural progenitor cells isolated from the adult human hippocampus." Nat. Med. 6:271-77).

Various therapies have so far been tried for such central nerve system diseases as spinal cord injury, Parkinson's disease and Alzheimer' s disease. However, any therapy leading to complete recovery from these diseases has not yet been found out. A regenerative therapy applicable to the central nerve system and a drug capable of inducing differentiation of nerve stem cells into functioning nerve cells, if available, will bring about a radical therapy for such central nerve system diseases. For realizing such therapy or drug, it is essential to isolate nerve stem cells using some or other marker. Several nerve stem cell marker genes, for example the Nestin, Musashi 1 and α-tubulin genes, have already been identified in the central nerve system (Keyoung, H. M. et al. (2001) "High-yield selection and extraction of two promoter-defined phenotypes of neural stem cells from the fetal brain." Nat. Biotech. 19:843-50; and Miller, F. et al. (1987) "Isotypes of alpha-tubulin are differentially regulated during neural maturation." J. Cell. Biol. 105:3065-73). Fundamentally, it is possible to use these genes to identify nerve stem cells/progenitor cells; however, each of those genes has both merits and demerits in its function as a marker.

### SUMMARY OF THE INVENTION

It is an object of the present invention to find out a novel nerve stem cell marker and provide a method for detecting nerve stem cells/progenitor cells among a tissue or a mass of cells as constituted of a plurality of cell species and, further, provide a method for sorting out nerve stem cells/progenitor cells.

### DETAILED DESCRIPTION OF THE INVENTION

For solving the above problems, the present inventors paid their attention to the NC1 gene. The NC1 gene, though first identified as a gene specifically expressed in the pancreas of patients with persistent hyperinsulinemic hypoglycemia of infancy (PHHI), has been shown to be a gene specifically expressed in the brain among normal tissues. The present inventors investigated the functions of the NC1 gene in further detail and found, by using the techniques described below, that this gene functions as a means for detecting nerve stem cells/progenitor cells.

Nerve stem cells are defined as undifferentiated nerve system cells capable of proliferating and being repeatedly passaged (self-replicating ability) and, at the same time, capable of generating cells, such as neurons and glial cells, which constitute the central nerve system (multipotency). A selective method (neurosphere method) for cultivating nerve stem cells/progenitor cells has been established by Weiss et al. (Science, 255, 1707 (1992)). According to the method of Weiss et al., a group of central nerve system cells, including nerve stem cells/progenitor cells, obtained from the murine embryonic spinal cord or the corpus striatum are cultured in a serum-free medium supplemented with EGF (epidermal growth factor) or FGF2 (fibroblast growth factor 2). On that occasion, many cells are damaged in the serum-free environment but nerve stem cells/progenitor cells proliferate under the culture conditions and form floating cell agglomerates (neurospheres).

The present inventors have already established a system for forming neurospheres from cells prepared from the fetal human spinal cord or the forebrain and cultivating the same in the presence of serum for differentiation thereof into neurons or glial cells. The expression of the NC1 gene was investigated using this system and, this time, it was found for the first time, that the expression thereof is high in the state of neurospheres, namely in nerve stem cells/progenitor cells and the expression decreases with an advance of differentiation into neurons or glial cells, as described hereinafter. Based on this finding, the present invention has been completed which makes it possible to detect nerve stem cells/progenitor cells and/or sorting nerve stem cells/progenitor cells utilizing the NC1 gene as a novel nerve stem cell marker and using the expression of the NC1 gene as an indicator.

Thus, the present invention relates to a DNA comprising one of the base sequences shown under SEQ ID NO:1 to SEQ ID NO:3 which can be used for the detection of the expression of the NC1 gene. Such DNA is a DNA comprising the whole or a part of the base sequence shown under SEQ ID NO:1; or a DNA comprising the base sequence shown under SEQ ID NO:2 or SEQ ID NO:3.

The invention also relates to a peptide comprising one of the amino acid sequences shown under SEQ ID NO:4 to SEQ ID NO:6 which serves as an epitope against an antibody specifically reacting with the NC1 gene product and capable of being used for the detection of the expression of the NC1 gene. The invention thus relates to a peptide comprising the amino acid sequence shown under SEQ ID NO:4; a peptide comprising the amino acid sequence shown under SEQ ID NO:5; a peptide comprising the amino acid sequence shown under SEQ ID NO:6; an antibody obtained by using one of the peptides mentioned above as an antigen; the antibody mentioned above which is a polyclonal antibody; the antibody mentioned above which is a monoclonal antibody.

The invention further relates to a method for detecting the expression of the NC1 gene by using at least one species selected from the group consisting of the above-mentioned DNAs, peptides and antibodies and to a method for screening for a drug functioning as a nerve differentiation factor or the like by using the expression of the NC1 gene as an indicator. Thus, the invention provides a method for detecting nerve stem cells/progenitor cells which uses at least one species selected from the group consisting of the above-mentioned DNAs, peptides and antibodies; and a method for screening for a drug functioning as a nerve differentiation factor or a nerve differentiation inhibitor which uses the above-mentioned detection method.

First, the method for detecting nerve stem cells/progenitor cells according to the invention is described.

The method for detecting nerve stem cells/progenitor cells according to the invention uses at least one of DNAs comprising one of the base sequence shown under SEQ ID NO:1 to 3, peptides comprising one of the amino acid sequences shown under SEQ ID NO:4 to SEQ ID NO:6, and antibodies obtained by using such the peptide as an antigen.

The nerve stem cells/progenitor cells, which are the targets of the present invention, are not particularly restricted with respect to the animal species, morphology, developmental stage and the like, provided that they can differentiate into functional nerve cells, such as neurons or glial cells, in vitro or in vivo. Mammal-derived cells are desirable and, from the clinical application viewpoint, human-derived ones are more desirable.

The Northern blotting, for instance, is available as a method for detecting the expression of a target gene in a certain tissue or cells.

As described later herein, the NC1 gene is highly expressed in nerve stem cells/progenitor cells and, therefore, it is possible to detect nerve stem cells/progenitor cells in a living tissue or a mass of cells as constituted of a plurality of cell species using the Northern blotting with the DNA comprising the whole or a part of the base sequence shown under SEQ ID NO:1, for instance, as a probe. The DNA comprising the base sequence shown under SEQ ID NO:1 can be obtained by PCR, for instance.

The DNA to be used as a probe is not limited to the DNA comprising the base sequence shown under SEQ ID NO:1 but all DNAs specifically recognizing the NC1 gene-derived mRNA can be used each as a probe.

The substance for labeling the probe includes, but is not particularly limited to, radioactive substances such as ³²P and ³⁵S; enzymes such as alkaline phosphatase and horseradish peroxidase; fluorescent substances such as fluorescein isothiocyanate; and the like.

The method for labeling the probe includes, but is not particularly limited to, an enzymatic binding method and a chemical binding method and, further, such in vitro transcription-based method as used in Example 1, as described later herein.

The method for detecting the expression of a target gene is not limited to the Northern blotting but, for example, RT-PCR or the primer extension technique may also be used for the same purpose.

When RT-PCR or the primer extension method is employed, a DNA comprising the base sequence shown under SEQ ID NO:2 and a DNA comprising the base sequence shown under SEQ ID NO:3 can be used as primers. The DNAs comprising the base sequence shown under SEQ ID NO:2 and 3, respectively, can be obtained by a DNA synthesis.

The primers to be used are not limited to those but all DNAs useful in amplifying a DNA fragment comprising a base sequence regarded as an NC1 gene-derived base sequence or specifically recognizing the NC1 gene transcript can be utilized as primers.

Further available as a method for detecting an occurrence of the target protein in a certain cell species is the Western blotting, for instance. It is possible, however, to detect nerve stem cells/progenitors cells among a living tissue or a mass of cells as constituted of a plurality of cell species by using an antibody specifically reacting with the NC1 gene product.

For producing such an antibody, it is necessary to prepare a protein or a peptide to serve as an antigen. The NC1 gene product is a protein composed of 247 amino acid residues, and the production of the protein to serve as the antigen requires a somewhat complicated production process even if genetic engineering techniques are utilized. Therefore, the present inventors decided to use a peptide, which can be prepared with ease, as an antigen and selected those peptides which comprise the amino acid sequences shown under SEQ ID NO:4 to SEQ ID NO:6 as the peptides comprising an amino acid sequence possibly serving as epitopes in view of the hydrophobicity and basicity of their amino acids.

The antigens to be used for producing antibodies reacting with the NC1 gene product are not limited to the peptides described herein but the whole or a part of the NC1 gene product composed of 247 amino acid residues may also be used.

Furthermore, for facilitating the antigen production and purification, it is also possible to employ, as the antigen, a protein or peptide further containing an amino acid sequence other than the NC1 gene product, for example the FLAG or Myc peptide, which can function as a tag sequence.

While, in an embodiment of the invention, the peptides used as antigens, for example the peptides comprising the amino acid sequences shown under SEQ ID NO:4 to SEQ ID NO:6, were synthesized by the solid method using a peptide synthesizer, a method or a mode of synthesis, an apparatus therefor and the like, are not particularly restricted.

The antibody reacting with the above NC1 gene product is not particularly restricted but may be a polyclonal antibody or a monoclonal antibody. Those methods known in the art can be used in producing the polyclonal and monoclonal antibodies.

In addition to the Western blotting, there are further available the histoimmunostaining method, the immunoprecipitation method and the like methods for the detection of the occurrence of the target protein. These methods are also usable in detecting nerve stem cells/progenitor cells among a living tissue or a mass of cells as constituted of a plurality of cell species using an antibody recognizing the NC1 gene product.

The screening method of the present invention is now described.

The method for screening for a drug functioning as a nerve differentiation factor or a nerve differentiation inhibitor according to the invention uses the above-mentioned method for detecting nerve stem cells/progenitor cells.

Thus, by utilizing the above method for detecting nerve stem cells/progenitor cells according to the invention, it is possible to screen for the drug functioning as the nerve differentiation factor, the nerve differentiation inhibitor or the like, for example, by the following method.

Neurospheres comprising nerve stem cells/progenitor cells, upon being stimulated with serum or the like, differentiate into nerve cells, such as neurons and glial cells. It is possible to screen for the drug having the desired activity by adding, to such a nerve cell differentiation-causing culture system, a substance, which is a candidate for the drug functioning as a nerve differentiation factor, a nerve differentiation inhibitor or the like, or an extract or the like possibly containing such a substance and evaluating the number of nerve stem cells contained in the culture system, the intensity of the NC1 gene expression serving as a nerve stem cell marker, or the like by using the above detection method in the present invention.

As for the nerve cell differentiation-causing culture system, a neurosphere culture system is most appropriate. However, any of other culture systems in which the level of the NC1 gene expression changes as the progress of differentiation may be used without any particular limitation.

The substance serving as the candidate for the drug functioning as a nerve differentiation factor or a nerve differentiation inhibitor, or the extract or the like possibly containing such a substance includes, but is not limited to, chemically synthesized compounds, microbial or cell culture fluids, extracts from individuals, tissues of organisms and the like.

The NC1 gene and NC1 protein of the present invention are considered to be involved in the process of differentiation of nerve system cells and can be applied as nerve stem cell markers with which nerve stem cells/progenitor cells can be detected or sorted out.

### BRIEF DESCRIPTION OF THE DRAWINGS

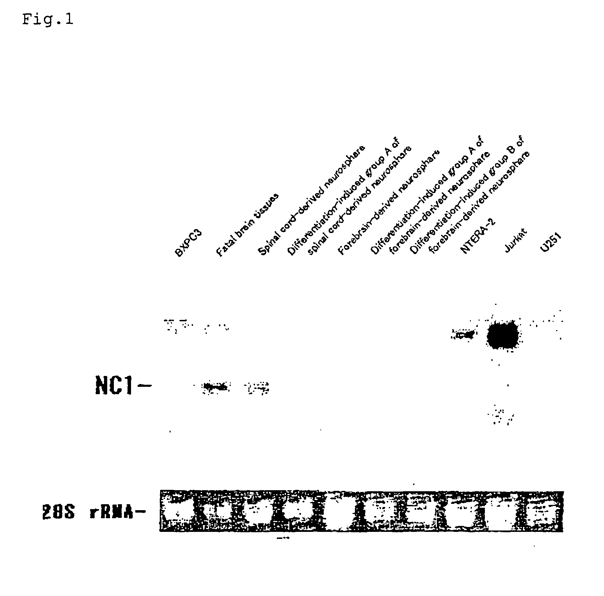

Fig. 1 is a representation of the results of the NC1 gene expression analysis by the Northern blotting. "NC1" indicates the position corresponding to the mobility of the NC1 gene-derived mRNA.

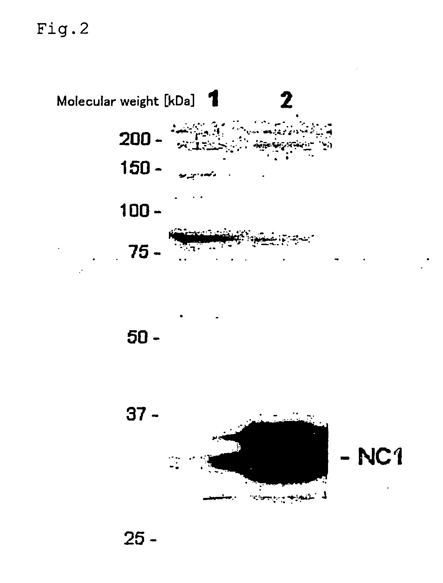

Fig. 2 is a representation of the results of detection, by the Western blotting, of the NC1 gene product forcedly expressed in Jurkat cells. In Lane 1, the Jurkat cell-derived whole RNA was electrophoresed and, in Lane 2, the whole RNA derived from Jurkat cells in which the NC1 gene had been forcedly expressed was electrophoresed.

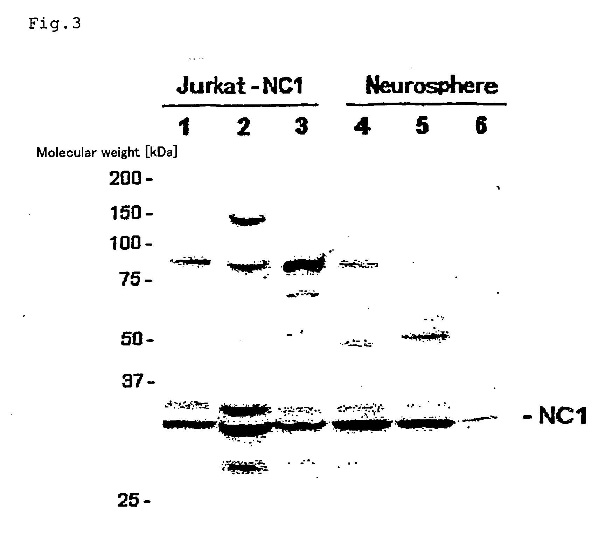

Fig. 3 is a representation of the results of detection, by the Western blotting, the NC1 gene product forcedly expressed in Jurkat cells and the NC1 gene product occurring in the neurospheres. In Lanes 1 and 4, the whole cell extracts were electrophoresed; in Lanes 2 and 5, the cytoplasmic fractions were electrophoresed; and, in Lanes 3 and 6, the nuclear fractions were electrophoresed.

### BEST MODE FOR CARYYING OUT THE INVENTION

The following examples illustrate the present invention more specifically. They are, however, by no means limitative of the scope of the invention. It is to be noted that various variations and modifications can be made without departing from the spirit of the invention.

### (Example 1) Detection of the NCI gene expression by the Northern blotting

The following experiments were carried out for detecting the expression of the NC1 gene by the Northern blotting.

### (1) Preparation of a probe for detection

The probe to be used in detecting the gene expression was constructed in an in vitro transcription system using a vector containing a DNA fragment corresponding to the NC1 gene as inserted therein. Specifically, the following procedure was followed. The oligonucleotides resulting from addition of an XhoI site to each of the base sequence shown under SEQ ID No: 2 and the base sequence shown under SEQ ID No: 3 were used as primers. A part of a human embryonic cDNA library obtained from Clontech Laboratories, Inc. was subjected to heat treatment, and the supernatant obtained by separating the sediment by centrifugation was used as the template. Using the above-mentioned primers and template, the DNA fragment containing an NC1 gene-specific sequence was amplified by PCR. The amplification product was cleaved with XhoI, followed by subcloning into a vector (pGEM-T Easy, product of Promega K.K.) . This vector thus contained the base sequence shown under SEQ ID NO:1 as a probe fragment. Using this vector, an RNA probe labeled with DIG-11-UTP (product of Roche Molecular Biochemicals) was prepared in an in vitro transcription system.

### (2) Cultivation and induced differentiation of nerve stem cells/progenitor cells

Tissue sections were collected from the human embryo forebrain and spinal cord, and neurospheres comprising human nerve stem cells/progenitor cells were cultivated using the neurosphere cultivation technique (Reynolds, B. A. et al., (1992) J. Neurosci, 12:4565-74; Vescovi, A. L. et al., (1993) Neuron 11:951-66; Reynolds, B. A. and Weiss, S., (1996) Dev. Biol. 175:1-13). As for the details of the cultivation technique, the procedure of Kanemura et al. (Kanemura, Y. et al., (2002) J. Neurosci. Res. 69:869-79) was followed.

In each of cell culture flasks with a base area of 75 cm², 3 x 10⁶ cells were precultured for 4 days and, then, cells were recovered by centrifugation and resuspended in a medium supplemented with retinoic acid and 1% of fetal calf serum. On the 7th day after the start of cultivation, the medium was exchanged for a fresh one and the cultivation was continued until the 14th day. This group of cells was designated as "differentiation-induced group A". The group of cells obtained by growing, similarly after 4 days of precultivation, the cells in a medium supplemented with fetal calf serum to a final concentration of 10% and continuing the cultivation until a confluent state after two passages was designated as "differentiation-induced group B".

### (3) Northern blotting

Total RNA was extracted from neurosphere-constituting cells, and from each of differentiation-induced groups A and B. 1-µg portion of each extract was developed on an agarose gel containing 1% of formamide, followed by transfer to a nylon membrane (Hybond-N; product of Amersham Biosciences K.K.). The membrane with transferred RNA and the labeled RNA probe prepared as described above in (1) were subjected to overnight hybridization in DIG Easy Hyb Buffer (product of Roche Molecular Biochemicals) at 68°C. After hybridization, the membrane was washed and then reacted with Anti-Digoxigenin-AP Fab Fragment (product of Roche Molecular Biochemicals) and subjected to the NC1 gene expression detection using CDP-Start chemiluminescent substrate (product of Amersham Biosciences K.K.).

### (4) Results

Using the materials and methods described above under (1) to (3), the expression of the NC1 gene was investigated in human nerve stem cells/progenitor cells and in each of the differentiation-induced groups of cells. Fig. 1 shows the results of the analyses. In Fig. 1, "NC1" indicates the position corresponding to the mobility of the NC1 gene-derived mRNA, and "28S rRNA" indicates the position corresponding to the mobility of the 28S ribosome RNA. "BXPC3" indicates the human pancreatic carcinoma-derived cell line BXPC3, "NTERA-2" the human embryonal carcinoma-derived cell line NTERA-2, "Jurkat" the human T cell line Jurkat, and "U251" the human glioblastoma-derived cell line U251.

The neurospheres prepared from the fetal human tissues comprise nerve stem cells/progenitor cells. In the differentiation-induced group A, the cells are composed of tubulin B III-positive cells having the characters of neurons and GFAP (glial fibrillary acidic protein)-positive cells having the characters of astrocytes and, in the differentiation-induced group B, almost all cells are GFAP-positive cells. In these culture systems, the expression of the NC1 gene was markedly high in neurospheres, and only low levels of the expression were observed in both the differentiation-induced groups A and B (Fig. 1). These results indicate that the expression of the NC1 gene is high in nerve stem cells/progenitor cells and decreases with the progress of differentiation into neurons and/or astrocytes. Thus, it was shown that since the NC1 gene highly expresses specifically in nerve stem cells/progenitor cells, this gene can be applied as a nerve stem cell marker.

### (Example 2) Detection of the NC1 gene product by Western blotting

The following experiments were carried out for detecting the NC1 gene product by the Western blotting.

### (1) Production of an antibody against the NC1 gene product

An antibody responsive to the NC1 gene product was produced by using, as an antigen, a partial peptide of the protein encoded by the NC1 gene. In practice, a partial peptide of the NC1 gene product, namely a peptide having the amino acid sequence shown under SEQ ID NO:4, was synthesized, and a rabbit was immunized therewith to give antiserum. It was confirmed by ELISA that this antiserum reacts with the original peptide at a titer of at least ten thousand times. Thus, this was used as antiserum containing an antibody against the NC1 gene product.

### (2) Preparation of test cells and cell extracts

An attempt was made to detect, by the Western blotting using antiserum prepared as described above under (1), the NC1 gene product in the neurospheres prepared from the fetal human nerve tissue in which the expression of the NC1 gene was detected in Example 1. Attempts were also made to detect, by the Western blotting, the NC1 gene product in Jurkat cells in which the expression of the NC1 gene was not detected and in a transformant strain derived from Jurkat cells by introduction of the NC1 gene expression vector. Whole cell extracts were prepared from these cell species and, further, cytoplasmic fractions and nuclear fractions were prepared using N-XTRACT Kit (product of Sigma).

### (3) Western blotting

Each sample prepared as described above in (2) was heated at 95°C for 5 minutes and then fractionated by SDS polyacrylamide gel electrophoresis, followed by transfer to a nitrocellulose membrane. The membrane was blocked with a blocking solution (Tris buffer containing 5% of skimmed milk and 0.1% of Tween 20) and, then, antiserum prepared as described above in (1) was added. The membrane was washed and then reacted with a horseradish peroxidase-labeled anti-rabbit IgG antibody (product of Amersham Biosciences K.K.) for detecting any reactive fraction using ECL (product of Amersham Biosciences K.K.).

### (4) Results

Using the materials and methods described above in (1) to (3), attempts were made to detect the NC1 gene product by means of antiserum prepared. The results are shown in Figs. 2 and 3. Referring to Fig. 2, the Jurkat cell-derived whole RNA was electrophoresed in Lane 1, and the whole RNA derived from Jurkat cells in which the NC1 gene had been forcedly expressed was electrophoreses in Lane 2. Referring to Fig. 3, in Lane 1, the whole cell extract derived from Jurkat cells in which the NC1 gene had been forcedly expressed was electrophoresed; in lane 2, the cytoplasmic fraction derived from Jurkat cells in which the NC1 gene had been forcedly expressed was electrophoresed; in Lane 3, the nuclear fraction derived from Jurkat cells in which the NC1 gene had been forcedly expressed was electrophoresed; in Lane 4, the whole cell extract from the neurospheres was electrophoresed; in Lane 5, the cytoplasmic fraction of the neurospheres was electrophoresed; and, in Lane 6, the nuclear fraction of the neurospheres was electrophoresed.

The antiserum mentioned above did not react with the Jurkat cells incapable of expressing the NC1 gene (Fig. 2, lane 1) but reacted with the transformant (Jurkat-NC1) derived from Jurkat cells and forcedly caused to express the NC1 gene, giving a band corresponding to the NC1 gene product (Fig. 2, lane 2). This indicates that antiserum produced as described above under (1) recognizes the NC1 gene product and that the product can be detected at least by the Western blotting.

When the fetal human nerve tissue-derived neurospheres were subjected to Western blotting, bands reacting with antiserum were detected at the same position as that detected in Jurkat-NC1 forcedly caused to express the NC1 gene (Fig. 3) . Furthermore, in the case of Jurkat NC1, antiserum reacted with both the cytoplasmic and nuclear fractions but, in the case of the neurospheres, the cytoplasmic fraction alone showed reactivity. Therefore, the occurrence of the NC1 gene product in nerve stem cells/progenitor cells, such as neurospheres, can be detected by Western blotting using this antiserum. Since this antiserum reacts not only with the whole cell extract but also with the fractionated extract, it is also possible to reveal the intracellular localization of the NC1 gene product.

### INDUSTRIAL APPLICABILITY

The NC1 gene and the NC1 protein of the invention are considered to be involved in the process of differentiation of nerve system cells and can be applied as nerve stem cell markers with which nerve stem cells/progenitor cells can be detected or sorted out.

## Claims

1. A DNA which comprises the whole or a part of the base sequence shown under SEQ ID NO:1.

2. A DNA which comprises the base sequence shown under SEQ ID NO:2 or SEQ ID NO:3.

3. A peptide which comprises the amino acid sequence shown under SEQ ID NO:4.

4. A peptide which comprises the amino acid sequence shown under SEQ ID NO:5.

5. A peptide which comprises the amino acid sequence shown under SEQ ID NO:6.

6. An antibody which is obtained by using one of the peptides according to any one of Claims 3 to 5 as an antigen.

7. The antibody according to Claim 6
wherein the antibody is a polyclonal antibody.

8. The antibody according to Claim 6
wherein the antibody is a monoclonal antibody.

9. A method for detecting nerve stem cells/progenitor cells which uses at least one species selected from the group consisting of the DNAs according to Claims 1 and 2, peptides according to Claims 3 to 5 and antibodies according to Claims 6 to 8.

10. A method for screening for a drug functioning as a nerve differentiation factor or nerve differentiation inhibitor using the method according to Claim 9.


SEQUENCE LISTING

<110> KANEKA CORPORATION
National Institute of Advanced Industrial Science and Technology
Foundation for Biomedical Research and Innovation

<120> Novel nerve stem cell marker

<130> B030361W001

<150> JP 2003-321564
<151> 2003-9-12

<160> 6

<210> 1
<211> 749
<212> DNA
<213> Homo sapiens

<220>
<223> NC1 probe

<400> 1
ggagcttggt aatgcaggtg gtgaaggagc aggttatgag agcacttaca accaagccta 60
gctccctgga ccagttcaag agcaaactgc agaacctgag ctacactgag atcctgaaaa 120
tccgccagtc cgagaggatg aaccaggaag atttccagtc ccgcccgatt ttggaactaa 180
aggagaagat tcagccagaa atcttagagc tgatcaaaca gcaacgcctg aaccgccttg 240

tggaagggac ctgctttagg aaactcaatg cccggcggag gcaagacaag ttttggtatt 300
gtcggctttc gccaaatcac aaagtcctgc attacggaga cttagaagag agtcctcagg 360
gagaagtgcc ccacgattcc ttgcaggaca aactgccggt ggcagatatc aaagccgtgg 420
tgacgggaaa ggactgccct catatgaaag agaaaggtgc ccttaaacaa aacaaggagg 480
tgcttgaact cgctttctcc atcttgtatg actcaaactg ccaactgaac ttcatcgctc 540
ctgacaagca tgagtactgt atctggacag atggactgaa tgcgctactc gggaaggaca 600
tgatgagcga cctgacgcgg aatgacctgg acaccctgct cagcatggaa atcaagctcc 660
gcctcctgga cctggaaaac atccagatcc ctgacgcacc tccgccgatt cccaaggagc 720
ccagcaacta tgacttcgtc tatgactgt 749

<210> 2
<211> 21
<212> DNA
<213> Artificial Sequence

<220>
<223> NC1 forward primer

<400> 2
ggagcttggt aatgcaggtg g 21

<210> 3
<211> 21
<212> DNA
<213> Artificial Sequence

<220>
<223> NC1 reverse primer

<400> 3
acagtcatag acgaagtcat a 21

<210> 4
<211> 19
<212> PRT
<213> Artificial Sequence

<220>
<223> NC1 epitope peptide

<400> 4
Ala Pro Pro Pro Ile Pro Lys Glu Pro Ser Asn Tyr Asp Phe Val Tyr
1               5                   10                  15
Asp Cys Asn

<210> 5
<211> 16
<212> PRT
<213> Artificial Sequence

<220>
<223> NC1 epitope peptide

<400> 5
Cys Pro His Met Lys Glu Lys Gly Ala Leu Lys Gln Asn Lys Glu Val
1               5                   10                  15

<210> 6

<211> 16

<212> PRT

<213> Artificial Sequence

<220>

<223> NC1 epitope peptide

<400> 6

Phe Arg Lys Leu Asn Ala Arg Arg Arg Gln Asp Lys Phe Trp Tyr Cys

1               5                   10                  15